# EUROPÄISCHE PATENTSCHRIFT

(11) **EP 2 776 222 B1**
(45) Veröffentlichungstag und Bekanntmachung des Hinweises auf die Patenterteilung: **03.02.2016**
(21) Anmeldenummer: 12790421.7
(22) Anmeldetag: 09.11.2012
(51) Int. Cl.: B26D 7/26

(54) **ULTRASCHALL-SCHNEIDEVORRICHTUNG**
ULTRASOUND CUTTING DEVICE
DISPOSITIF DE COUPE À ULTRASONS

(30) Priorität: 11.11.2011 DE 102011118208
(43) Veröffentlichungstag der Anmeldung: 17.09.2014
(73) Patentinhaber: Artech Ultrasonic Systems AG, 8598 Bottighofen (CH)
(72) Erfinder: KISING, Jürgen, CH-8280 Kreuzlingen (CH)
(74) Vertreter: Dimmerling & Huwer
(86) Internationale Anmeldenummer: PCT/EP2012/004663
(87) Internationale Veröffentlichungsnummer: WO 2013/068123

(56) Entgegenhaltungen:
- DE-A1- 4 310 832
- DE-A1- 4 421 465
- DE-A1-102007 014 635
- JP-A- 7 266 297

## Beschreibung

Die Erfindung betrifft eine Ultraschall-Schneidevorrichtung mit mindestens einem mit einem Generator verbundenen Ultraschallkonverter, wenigstens einem Schallleiter und zumindest einer Schneidklinge, wobei der Schallleiter zwischen dem Ultraschallkonverter und der Schneidklinge angeordnet ist und diese miteinander verbindet, und wobei die Längsmittelachse des Schallleiters einen von einer geraden Linie abweichenden Verlauf aufweist.

Eine derartige Ultraschall-Schneidevorrichtung zum Schneiden von Lebensmitteln, wie z.B. Teigwaren, Käse, Fisch oder dergleichen Schneidgut, ist aus DE 43 10 832 A1 bekannt. Die Schneidevorrichtung weist eine flächige, sich im Wesentlichen in einer Ebene erstreckende Schneidklinge auf, die an ihrem von der Messerspitze entfernten Ende einstückig mit einem Schallleiter verbunden ist. Ein von der Schneidklinge beabstandeter Abschnitt des Schallleiters ist mit einem Ultraschallkonverter verschraubt, mittels dem in Längserstreckungsrichtung der Schneidklinge Ultraschallschwingungen in den Schallleiter einkoppelt werden können. Zwischen dem Ultraschallkonverter und der Schneidklinge weist der Schallleiter in einer normal zur Erstreckungsebene der Schneidklinge verlaufenden, sich in Längsrichtung der Schneidklinge erstreckenden Ebene eine 90°-Krümmung mit einem vorgegebenen Krümmungsradius auf. Der gekrümmte Verlauf geht an dem mit der Schneidklinge verbundenen Ende stetig differenzierbar in die Schneidklinge über. Durch den von der geraden Linie abweichenden Verlauf des Schallleiters wird dieser sowohl in Längserstreckungsrichtung der Schneidklinge als auch in einer normal zu der von der Schneidklinge aufgespannten Ebene zu Schwingungen angeregt. Dabei bewirkt die normal zur Ebene der Schneidklinge orientierte Schwingungskomponente eine Reduzierung der Reibung zwischen den Messerflanken der Schneidklinge und dem Schnittgut. Dennoch tritt beim Schneiden noch eine gewisse Reibung auf, welche die Schnittgeschwindigkeit begrenzt. Auch hat sich herausgestellt, dass Schneidklinge durch die Ultraschallschwingungen an unterschiedlichen Stellen unterschiedlich starken mechanischen Belastungen ausgesetzt ist.

Aus DE 44 21 465 A1 ist ferner eine Ultraschall-Schneidevorrichtung bekannt, die ein Ultraschallgerät aufweist, das von einem HF-Generator angesteuert wird, der eine höherfrequente Wechselspannung zwischen 20 und 30 kHz erzeugt. In dem Ultraschallgerät wird die elektrische Wechselspannung in Ultraschall gleicher Frequenz umgewandelt. Die Ultraschall-Schneidevorrichtung weist eine Aktivteil mit einem sitzen Hammerende und ein passives, als Ambos dienendes Teil auf. Die Ultraschall-Schneidevorrichtung wirkt wie ein mit Ultraschall betriebener elektrischer Hammer, der zum Schneiden einer Gewebebahn genutzt werden kann.

Es besteht deshalb die Aufgabe, eine Ultraschall-Schneidevorrichtung der eingangs genannten Art zu schaffen, die einen einfachen Aufbau mit kompakten Abmessungen aufweist, aber dennoch eine hohe Schnittgeschwindigkeit und eine gleichmäßige mechanische Beanspruchung der Schneidklinge ermöglicht.

Erfindungsgemäß wird diese Aufgabe dadurch gelöst, dass der mindestens eine Generator Mittel zum Durchlaufen eines vorbestimmten Ultraschallfrequenzbereichs entsprechend einer Sweep-Funktion aufweist.

In vorteilhafter Weise sind die Knotenpunkte der Ultraschallwellen auf der Schneidklinge dann nicht ortsfest sondern verändern ihre Lage entsprechend der Frequenzvariation. Dadurch kann in den Knotenpunkten ein Anhaften der Schneidklinge am Schneidgut vermieden werden. Durch die Frequenzvariation wird außerdem die mechanische Belastung in der Schneidklinge reduziert, da große Resonanzamplituden nur kurzzeitig auftreten und dadurch eine impulsartige Anregung resultiert, die sich als vorteilhaft für das Schneiden herausgestellt hat, da dadurch das zu schneidende Gut abgeschüttelt wird. Die Frequenzvariation ermöglicht die Anregung unterschiedlichster Messergeometrien und den Einsatz von mehreren Ultraschallkonvertern an nur einem Generator, wodurch sehr breite Schnittmesser erzeugt werden können. Der von der geraden Linie abweichende Verlauf des Schallleiters kann derart gewählt sein, dass die Schneidklinge in quer zueinander verlaufenden Richtungen zu Ultraschallschwingungen anregbar ist. Dadurch können Plattenwellen in die Schneidklinge eingekoppelt werden, die sich zwischen im Wesentlichen parallel zueinander verlaufenden und/oder keilförmig zur Schneidfläche hin aufeinander zulaufenden Grenzflächen der Schneidklinge ausbreiten können. Die Plattenwellen weisen sowohl eine in der von der Schneidklinge aufgespannten Fläche quer zur Längserstreckung der Schneidklinge orientierte longitudinale Komponente aus auch eine quer zu dieser Fläche orientierte transversale Schwingungskomponente auf. Dies ist für das Schneiden vorteilhaft, da das Schneidgut einerseits einen seitlichen, quer zu der von der Schneidklinge aufgespannten Fläche ausgerichteten Impuls erhält, der zu einer besseren Ablösung des Schneidguts von der Schneidklinge führt, und die Schneidklinge andererseits aber auch in einer in der von ihr aufgespannten Fläche liegenden Richtung zum Schwingen angeregt wird, vorzugsweise in Längsrichtung der Schneidklinge. Dadurch wird während des Schneidvorgangs die Reibung reduziert, so dass die in die Schneidklinge eingekoppelte Ultraschallleistung besser für den Schneidvorgang genutzt werden kann. Durch die Plattenwellen kann die Schneidklinge vergleichsweise große Abmessungen aufweisen, ohne dass in der Schneidklinge Schlitze vorgesehen sein müssen. Dies ermöglicht ein kostengünstiges Messerdesign. Bezüglich der Geometrie der Schneidklinge bestehen praktisch keine Einschränkungen.

Aus DE 10 2007 014 635 A1 ist zwar bereits eine Vorrichtung zur Ultraschallanregung von Strukturen bekannt, die einen mit einem Generator verbundenen Ultraschallkonverter und Mittel zum Durchlaufen eines vorbestimmten Ultraschallfrequenzbereichs aufweist. Diese Vorrichtung weist jedoch keine Schneidklinge auf. Vielmehr dient die Vorrichtung dazu, mit einem Ultraschallkonverter mehrere Siebe, die voneinander abweichende Resonanzfrequenzen aufweisen, gleichzeitig anzuregen.

Der Schallleiter weist bevorzugt einen gekrümmten Verlauf auf. Dabei kann sich die Richtung, in die sich der Schallleiter erstreckt, um mindestens 45°, insbesondere um mindestens 60°, gegebenenfalls um mindestens 75° und bevorzugt um etwa 90° ändern.

Bei einer vorteilhaften Ausgestaltung der Erfindung ist die Schneidklinge als flächiges Element ausgestaltet, wobei der Schallleiter als Leitungsstab ausgebildet ist, der quer zu der Fläche, in der sich die Schneidklinge erstreckt, mit dieser verbunden ist. Die Plattenwellen können dadurch noch besser in der Schneidklinge angeregt werden. Die Schneidklinge kann parallel oder konzentrisch zueinander verlaufende Grenzflächen aufweisen und/oder Grenzflächen, die keilförmig aufeinander zulaufen.

Bei einer zweckmäßigen Ausführungsform der Erfindung ist der Schallleiter ringförmig ausgebildet, wobei ein erster Endbereich des Schallleiters mit dem Ultraschallkonverter und ein dem ersten Endbereich diametral gegenüberliegender zweiter Endbereich des Schallleiters mit der Schneidklinge verbunden ist. Dadurch ist es möglich, den Ultraschall symmetrisch von zwei Seiten in die Schneidklinge einzukoppeln. Der Schallleiter weist in der von ihm aufgespannten Ebene bevorzugt eine ovale oder eine kreisringförmige Geometrie auf. Er kann aber auch eckig ausgestaltet sein.

Die Schneidklinge kann im Wesentlichen zylindrisch ausgestaltet sein, wobei der Schallleiter an der Zylindermantelfläche der Schneidklinge mit dieser verbunden ist. Dabei wird unter einem zylindrischen Schallleiter ein flächiger Schallleiter verstanden, der sich in einer Fläche erstreckt, die durch Verschiebung einer in einer Ebene verlaufenden Kurve entlang einer Geraden, die nicht in dieser Ebene liegt, erzeugt werden kann. Die Gerade kann normal zu der Ebene (gerader Zylinder) oder schräg zur Ebene (schiefer Zylinder) verlaufen.

Bei einer zweckmäßigen Ausgestaltung der Erfindung ist die Schneidklinge kreiszylindrisch oder oval ausgebildet. Mit einer solchen Schneidklinge können beispielsweise aus einem Vollmaterial stabförmige Gegenstände ausgeschnitten werden.

Die Schneidklinge kann aber auch eckig ausgestaltet sein, insbesondere rechteckig. Mit einer solchen Schneidklinge können beispielsweise prismenförmige Gegenstände aus einem Schneidgut ausgeschnitten werden.

Bei einer vorteilhaften Ausführungsform der Erfindung weist die Ultraschall-Schneidevorrichtung mehrere Ultraschallkonverter auf, die jeweils über wenigstens einen Schallleiter mit voneinander beabstandeten Schalleinkoppelstellen der Schneidklinge verbunden sind. Dadurch kann bei größeren Schneidklingen ein Amplitudenabfall an der Schneidklinge vermieden werden.

Bei einer Weiterbildung der Erfindung ist der Schallleiter einstückig mit der Schneidklinge verbunden und vorzugsweise durch einen etwa U-förmig gekrümmten Messerschaft gebildet. Dadurch ergibt sich eine besonders einfach aufgebaute, robuste Ultraschall-Schneidevorrichtung.

Nachfolgend sind Ausführungsbeispiele der Erfindung anhand der Zeichnung näher erläutert. Es zeigt:
- Fig. 1: eine Vorderansicht einer Ultraschall-Schneidevorrichtung, die ein gerades Messer aufweist, in das mit Hilfe von mehreren Ultraschallkonvertern angeregt wird,
- Fig. 2: eine Seitenansicht der in Fig. 1 gezeigten Ultraschall-Schneidevorrichtung,
- Fig. 3: eine Ultraschall-Schneidevorrichtung, die ein kreiszylindrisches Messer aufweist,
- Fig. 4: eine Ultraschall-Schneidevorrichtung, die ein rechteckiges Messer aufweist,
- Fig. 5: eine Aufsicht auf eine Ultraschall-Schneidevorrichtung, bei der das Messer über einen ovalen Schallleiter mit einem Ultraschallkonverter verbunden ist,
- Fig. 6 und 7: Seitenansichten einer Ultraschall-Schneidevorrichtung, bei der an das Messer ein Schallleiter einstückig angeformt ist, und
- Fig. 8: eine Seitenansicht einer Ultraschall-Schneidevorrichtung, bei der die Ultraschallwellen von oben in das Messer eingekoppelt werden.

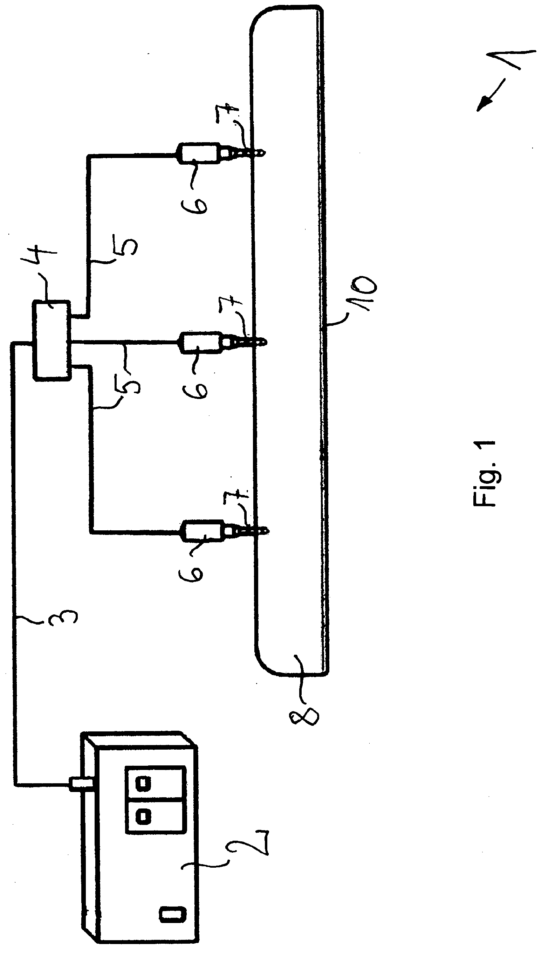

Eine in Fig. 1 im Ganzen mit 1 bezeichnete Ultraschall-Schneidevorrichtung hat einen Ultraschall-Generator 2, der Mittel zum Durchlaufen eines vorbestimmten Ultraschallfrequenzbereichs aufweist (Sweep-Funktion). Der Generator 2 ist über ein erstes Hochfrequenzkabel 3 mit einem Eingangsanschluss eines Verteilers 4 verbunden. Der Verteiler 4 hat drei Ausgangsanschlüsse, an denen jeweils über ein zweites Hochfrequenzkabel 5 ein Hochfrequenzeingang eines Ultraschallkonverters 6 angeschlossen ist.

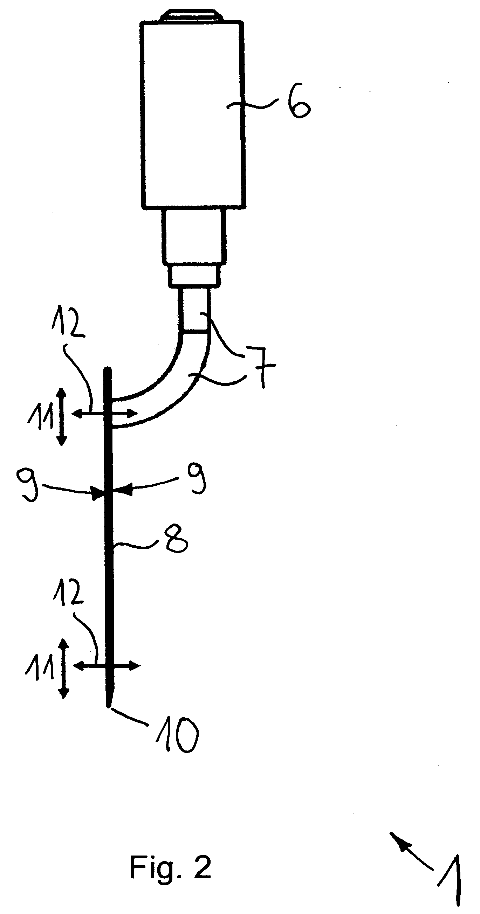

Jeder Ultraschallkonverter 6 ist jeweils über einen stabförmigen Schallleiter 7 mit einer ihm zugeordneten Einkoppelstelle einer Schneidklinge 8 verbunden. Wie in Fig. 1 und 2 erkennbar ist, ist die Schneidklinge 8 als dünne Platte ausgestaltet, die zwei parallel zueinander verlaufende Grenzflächen 9 hat, von denen die eine fest mit den Schallleitern 7 verbunden ist. An ihrem unteren Randbereich verjüngt sich die Schneidklinge 8 keilförmig zu einer Schneidkante 10 hin. Es sind aber auch andere Ausgestaltungen denkbar, bei denen die Schneidklinge 8 über ihre gesamte Höhe keilförmig ausgestaltet ist.

In Fig. 2 ist erkennbar, dass der Schallleiter 10 einen bogenförmig gekrümmten Abschnitt aufweist, der sich in einer normal zur Längsachse der Schneidklinge 8 angeordneten, parallel zur Zeichenebene von Fig. 2 verlaufenden Ebene erstreckt. Der bogenförmig gekrümmten Schallleiter-Abschnitt weist eine Krümmung von etwa 90° auf und ist an seinem der Grenzfläche 9 zugewandten Ende über eine in der Zeichnung nicht näher dargestellte Schweißnaht mit der Schneidklinge 8 verbunden. An seinem von der Schneidklinge 8 entfernten anderen Ende ist der bogenförmig gekrümmte Schallleiter-Abschnitt über einen geraden Schallleiter-Abschnitt mit dem ihm zugeordneten Ultraschallkonverter 6 verbunden. Dieser koppelt in einer normal zur Längsachse der Schneidklinge 8 und parallel zu dieser verlaufenden Richtung Ultraschallschwingungen in das von der Schneidklinge 8 entfernte Ende des Schallleiters 7 ein.

Erwähnt werden soll noch, dass auch andere von einer geraden Linie abweichende Verläufe des Schallleiters 7 denkbar sind, beispielsweise ein S- oder L-förmiger Verlauf des Schallleiters 7.

Durch die Ausgestaltung des Schallleiters 7 als gekrümmter Leiterstab wird die Schneidklinge 8 in einer normal zur Längsachse der Schneidklinge 8 angeordneten, der Zeichenebene in Fig. 2 entsprechenden Ebene sowohl in Richtung der Schnittlinie zwischen dieser Ebene und der Erstreckungsebene der Schneidklinge 8 entsprechend den Doppelpfeilen 11 als auch in einer normal zur Erstreckungsebene der Schneidklinge 8 orientierten, durch die Doppelpfeile 12 angedeuteten Richtung zu Schwingungen angeregt.

Zur Erfassung der von den Generatoren 2 an die Ultraschallkonverter 6 abgegebenen Leistung weist die Ultraschall-Schneidevorrichtung 1 eine in der Zeichnung nicht näher dargestellte Messeinrichtung auf. Diese steht über eine Steuereinrichtung mit den Mitteln zum Durchlaufen des vorbestimmten Ultraschallfrequenzbereichs in Steuerverbindung. Zunächst wird ein erster Scan durchgeführt, bei dem die Ultraschallfrequenz ausgehend von einem vorgegebene Startwert bis zu einem vorgegebenen Endwert verändert wird. Der Startwert kann beispielsweise etwa 30 kHz und der Endwert etwa 38 kHz betragen.

Beim Durchlaufen dieses Ultraschallfrequenzbereichs wird die vom Generator 2 abgegebene Leistung als Funktion der Ultraschallfrequenz gemessen. Danach wird mittels eines Mikroprozessors der Frequenzpunkt f₀ ermittelt, an dem die höchste Leistung abgegeben wird. Dieser Frequenzpunkt wird gespeichert. Dann werden der kleinste Frequenzwert fₘᵢₙ und der größte Frequenzwert fₘₐₓ eines Ultraschallfrequenzbands bestimmt, das mit einer einstellbaren Bandbreite von z. B. bis 4000 Hz vorzugsweise symmetrisch um diesen Frequenzpunkt f₀ herum liegt. Der kleinste Frequenzwert kann z.B. fₘᵢₙ = f₀-2000 Hz und der größte Frequenzwert fₘₐₓ = f₀+2000 Hz betragen.

Der Generator 2 wird zunächst so angesteuert, dass die Schneidklinge 8 mit dem kleinsten Frequenzwert fₘᵢₙ angeregt wird. Danach wird die Frequenz jeweils um einen vorbestimmten Betrag von z. B. 1 Hz erhöht, um die Schneidklinge 8 mit der jeweils so erhaltenen neuen Frequenz anzuregen.

Nach jedem Erhöhen der Frequenz wird geprüft, ob die neue Frequenz kleiner ist als der zuvor bestimmte größte Frequenzwert fₘₐₓ. Falls das der Fall ist, werden die vorgenannten Schritte, bestehend aus dem Erhöhen der Frequenz, dem Anregen der Schneidklinge 8 mit dieser Frequenz und dem Prüfen, ob die neue Frequenz kleiner ist als der größte Frequenzwert fₘₐₓ, wiederholt.

Falls die neue Frequenz nicht kleiner ist als der größte Frequenzwert fₘₐₓ, wird die Frequenz jeweils um den vorbestimmten Betrag reduziert, um die Schneidklinge 8 jeweils mit der so erhaltenen neuen Frequenz anzuregen.

Nach jedem Reduzieren der Frequenz wird geprüft, ob die neue Frequenz größer ist als der zuvor bestimmte kleinste Frequenzwert fₘᵢₙ. Falls das der Fall ist, werden die oben genannten Schritte, bestehend aus dem Reduzieren der Frequenz, dem Anregen der Schneidklinge 8 mit dieser Frequenz und dem Prüfen, ob die neue Frequenz größer ist als der kleinste Frequenzwert fₘₐₓ, wiederholt.

Falls die neue Frequenz nicht größer ist als der kleinste Frequenzwert fₘᵢₙ, werden die oben genannten Schritte, ausgehend von dem kleinsten Frequenzwert fₘᵢₙ, bei Bedarf erneut durchlaufen.

Der Anwender kann die Bandbreite, in der dieser Sweep abläuft, zwischen 200 Hz und 4000 Hz einstellen. Die Schrittweite für den Frequenzschritt kann auch mehr als 1 Hz betragen. Durch die Einstellung der Bandbreite kann das Schnittergebnis noch optimiert werden. Um ein Weglaufen des Resonanzpunktes durch Temperatureinfluss oder Koppelschwankungen zu kompensieren wird in regelmäßigen Zeitabständen einen Neuscan wie am Anfang beim Einschalten durchgeführt, um dem Resonanzpunkt f₀ zu folgen. Dieser Neuscan wird allerdings nicht mehr über den gesamten Bereich von 30 bis 38 KHz gemacht sondern nur noch unmittelbar um den Resonanzpunkt f₀, um keine Totzeiten zu generieren, da der Neuscan mit geringer Leistung durchgeführt wird.

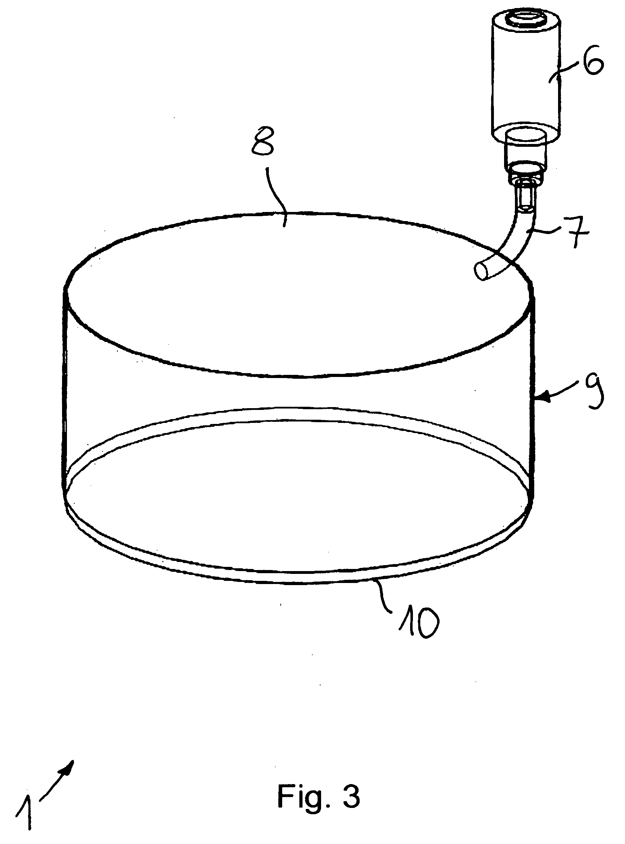

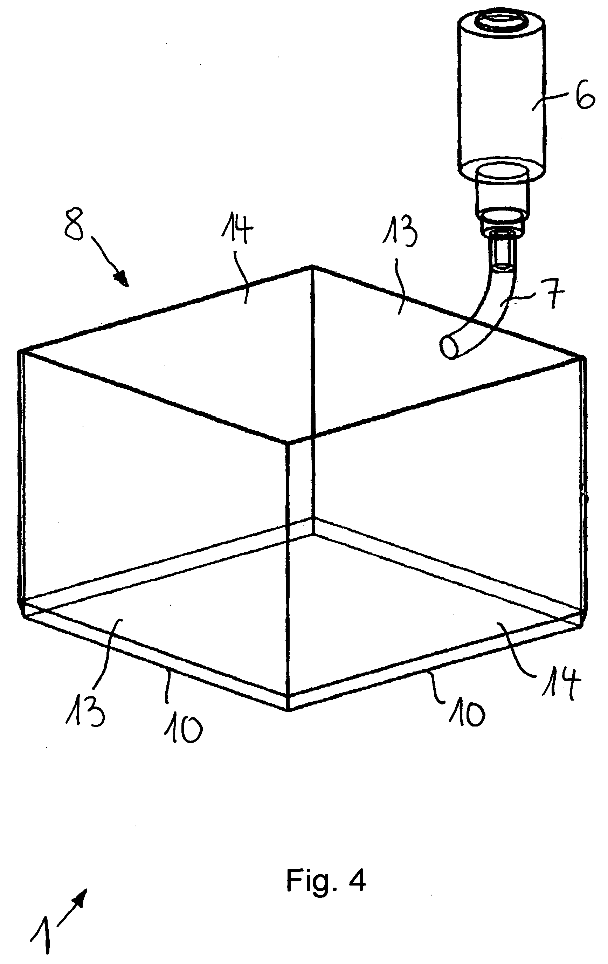

Bei den in Fig. 3 und 4 gezeigten Ausführungsbeispielen ist die Schneidklinge 8 im Wesentlichen zylindrisch ausgestaltet. Bei dem Ausführungsbeispiel gemäß Fig. 3 ist die Schneidklinge 8 durch ein dünnwandiges kreiszylindrisches Rohr gebildet, das sich an seinem unteren Randbereich zur Schneidkante 10 hin keilförmig verjüngt. Die Einkopplung des Ultraschalls erfolgt über einen gekrümmten Schallleiter 7, dessen Geometrie im Wesentlichen der des in Fig. 2 gezeigten Schallleiters 7 entspricht. Die Einkoppelstelle ist von der Schneidkante 10 beabstandet und befindet sich am oberen Randbereich der Schneidklinge 8. Die Einkoppelstelle kann aber auch an einer anderen Stelle der Schneidklinge 8 angeordnet sein, beispielsweise an dem zu der Schneidkante 10 benachbarten unteren Randbereich der Schneidklinge 8.

Bei dem in Fig. 4 gezeigten Ausführungsbeispiel hat die Schneidkante 10 einen rechteckigen Verlauf. Deutlich ist erkennbar, dass die Schneidklinge 8 zwei parallel zueinander angeordnete erste Schneidklingenabschnitte 13 und zwei quer dazu angeordnete, ebenfalls parallel zueinander verlaufende zweite Schneidklingenabschnitte 14 aufweist. Die ersten Schneidklingenabschnitte 13 und die zweiten Schneidklingenabschnitte 14 sind jeweils als dünne, ebene Platten ausgestaltet, die sich an ihrem unteren Randbereich zur Schneidkante 10 hin keilförmig verjüngen.

Die ersten Schneidklingenabschnitte 13 sind mit den zweiten Schneidklingenabschnitten 14 kastenförmig verbunden. Die Einkopplung des Ultraschalls erfolgt wiederum über einen gekrümmten Schallleiter 7, dessen Geometrie im Wesentlichen der des in Fig. 2 gezeigten Schallleiters 7 entspricht. Die Einkoppelstelle ist von der Schneidkante 10 beabstandet und befindet sich am oberen Randbereich der Schneidklinge 8.

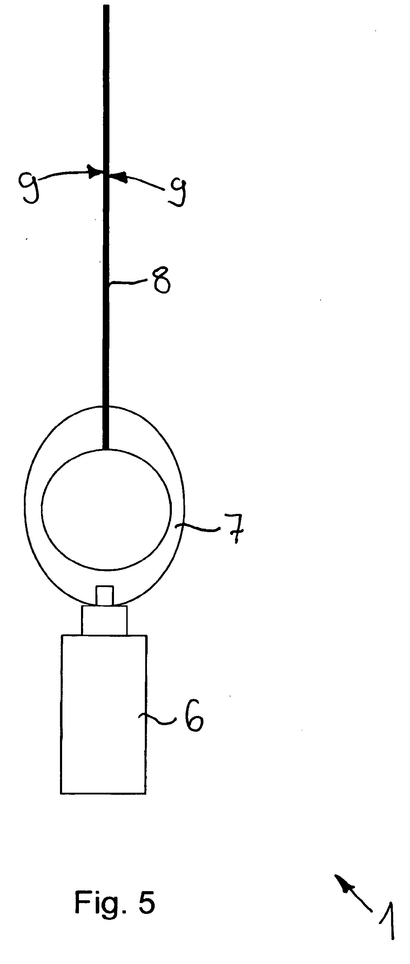

Bei dem in Fig. 5 gezeigten Ausführungsbeispiel ist der Schallleiter 7 oval ausgestaltet. Dabei ist die vom Schallleiter 7 aufgespannte Ebene rechtwinklig zu der Ebene ausgerichtet, in welcher der plattenförmige Schallleiter 7 angeordnet ist. Die Schneidkante 10 des Schallleiters 7 erstreckt sich im Wesentlichen parallel zu der vom Schallleiter 7 aufgespannte Ebene.

Ein erster Endbereich des Schallleiters 7 ist mit dem Ultraschallkonverter 6 und ein dem ersten Endbereich diametral gegenüberliegender zweiter Endbereich mit der Schneidklinge 8 verbunden. Der Ultraschallkonverter 6 ist in gerader Verlängerung der Schneidklinge 8 angeordnet und koppelt in Längsrichtung der Schneidklinge 8 Ultraschallwellen in den Schallleiter 7 ein.

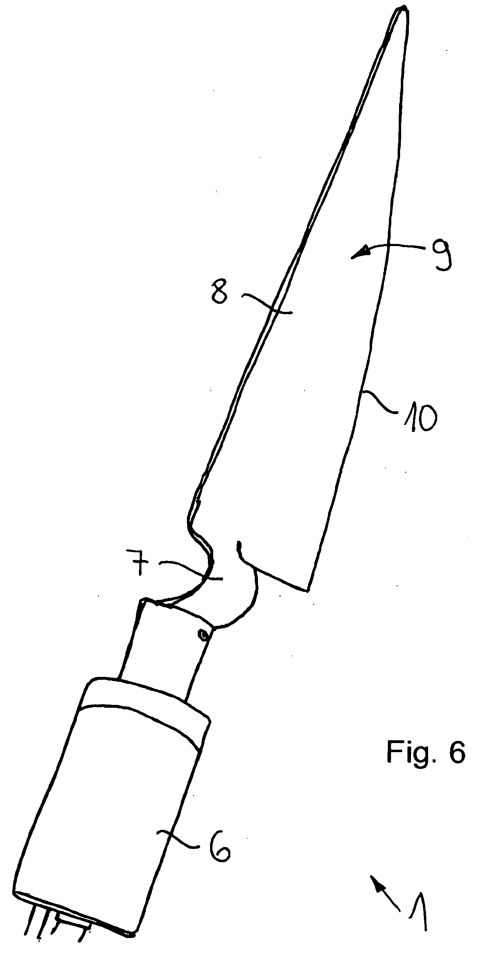

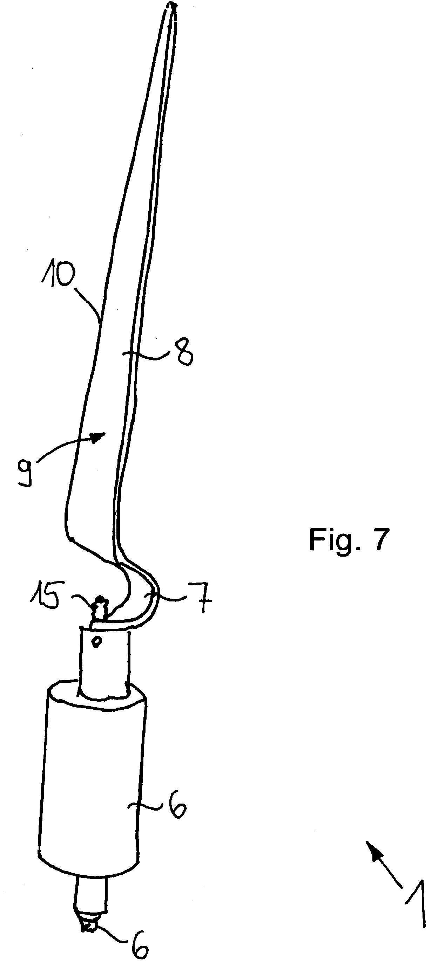

Bei dem in Fig. 6 und 7 abgebildeten Ausführungsbeispiel ist die Schneidklinge 8 als ebene dünne Platte ausgestaltet, die sich zur Schneidkante 10 hin vorzugsweise über die gesamte Höhe der Schneidklinge 8 keilförmig verjüngt. Die Höhe der Schneidklinge 8 nimmt, ausgehend von dem vom Schallleiter 7 entfernten Ende der Schneidklinge 8 zum Schallleiter 7 hin kontinuierlich zu.

Der Schallleiter 7 ist einstückig mit der Schneidklinge 8 ausgestaltet und durch den Messerschaft bzw. das Heft der Schneidklinge 8 gebildet. In einer rechtwinklig zur Erstreckungsebene der Schneidklinge 8 angeordneten, parallel zur Längsachse der Schneidklinge 8 verlaufenden Ebene hat der Messerschaft einen U-förmig Verlauf. Der Schallleiter 7 hat einen etwa rechteckigen Querschnitt. An seinem von der Schneidklinge 8 entfernten freien Ende ist der Schallleiter 7 mittels einer Schraube 15 lösbar mit dem Ultraschallkonverter 6 verbunden.

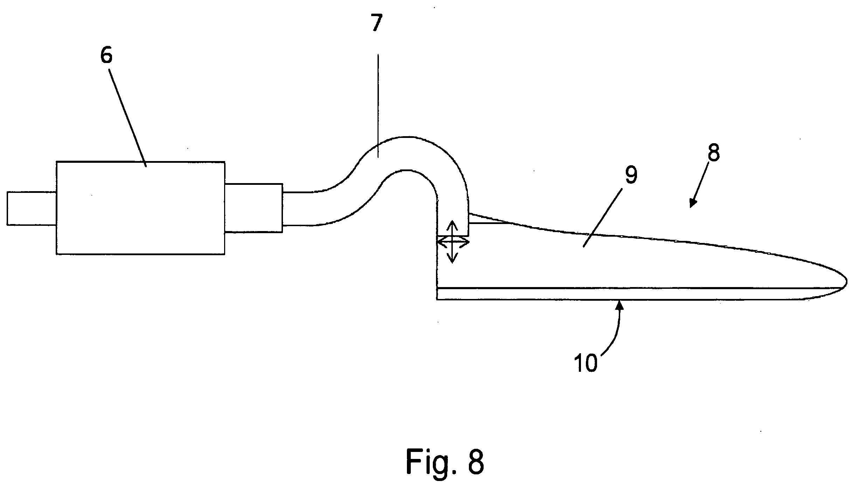

Bei dem in Fig. 8 gezeigten Ausführungsbeispiel ist die Schneidklinge 8 in ihrem der Schneidkante 10 abgewandten Rücken mit dem Schallleiter 7 verbunden. Die Ultraschallwellen werden also von oben in die Schneidklinge 8 eingekoppelt. Deutlich ist erkennbar, dass sich der der Schneidklinge 8 zugewandte Endbereich des Schallleiters 7 in etwa senkrecht zur Schneidkante 10 erstreckt. Dabei weist der Schallleiter 7 in der Eben, in der sich die plattenförmige Schneidklinge 8 erstreckt, bogenförmig in zueinander entgegen gesetzte Richtungen gekrümmt. Der Ultraschallkonverter 6 ist mit seiner Längsachse parallel zur Schneidkante 10 ausgerichtet.

## Patentansprüche

1. Ultraschall-Schneidevorrichtung (1) mit mindestens einem mit einem Generator (2) verbundenen Ultraschallkonverter (6), wenigstens einem Schallleiter (7) und zumindest einer Schneidklinge (8), wobei der Schallleiter (7) zwischen dem Ultraschallkonverter (6) und der Schneidklinge (8) angeordnet ist und diese miteinander verbindet, und wobei die Längsmittelachse des Schallleiters (7) einen von einer geraden Linie abweichenden Verlauf aufweist, **dadurch gekennzeichnet, dass** der mindestens eine Generator (2) Mittel zum Durchlaufen eines vorbestimmten Ultraschallfrequenzbereichs entsprechend einer Sweep-Funktion aufweist.

2. Ultraschall-Schneidevorrichtung (1) nach Anspruch 1, **dadurch gekennzeichnet, dass** sie eine Messeinrichtung zur Erfassung der vom Generator (2) an den Ultraschallkonverter (6) abgegebenen Leistung aufweist, und dass die Messeinrichtung derart mit den Mitteln zum Durchlaufen des vorbestimmten Ultraschallfrequenzbereichs in Steuerverbindung steht, dass der Ultraschallfrequenzbereich so gewählt wird, dass er die Ultraschallfrequenz beinhaltet, bei welcher der Generator (2) seine maximale Leistung an den Ultraschallkonverter (6) abgibt.

3. Ultraschall-Schneidevorrichtung (1) nach Anspruch 2, **dadurch gekennzeichnet, dass** die Messeinrichtung derart mit den Mitteln zum Durchlaufen des vorbestimmten Ultraschallfrequenzbereichs in Steuerverbindung steht, dass der Generator (2) seine maximale Leistung bei einer Ultraschallfrequenz an den Ultraschallkonverter (6) abgibt, die mittig zwischen der höchsten und der niedrigsten Frequenz des bei einem Frequenzdurchlauf durchlaufenen Ultraschallfrequenzbereichs liegt.

4. Ultraschall-Schneidevorrichtung (1) nach einem der Ansprüche 1 bis 3,
**dadurch gekennzeichnet, dass** die Schneidklinge (8) als flächiges Element ausgestaltet ist, und dass der Schallleiter (7) als Leitungsstab ausgebildet ist, der quer zu der Fläche, in der sich die Schneidklinge (8) erstreckt, mit dieser verbunden ist.

5. Ultraschall-Schneidevorrichtung (1) nach einem der Ansprüche 1 bis 4,
**dadurch gekennzeichnet, dass** der Schallleiter (7) ringförmig ausgebildet ist, dass ein erster Endbereich des Schallleiters (7) mit dem Ultraschallkonverter (6) und ein dem ersten Endbereich diametral gegenüberliegender zweiter Endbereich mit der Schneidklinge (8) verbunden ist.

6. Ultraschall-Schneidevorrichtung (1) nach Anspruch 5, **dadurch gekennzeichnet, dass** die Schneidklinge (8) im Wesentlichen zylindrisch ausgestaltet ist, und dass der Schallleiter (7) an der Zylindermantelfläche der Schneidklinge (8) mit dieser verbunden ist.

7. Ultraschall-Schneidevorrichtung (1) nach Anspruch 6, **dadurch gekennzeichnet, dass** die Schneidklinge (8) kreiszylindrisch oder oval ausgebildet ist.

8. Ultraschall-Schneidevorrichtung (1) nach Anspruch 6, **dadurch gekennzeichnet, dass** die Schneidklinge (8) eckig ausgestaltet ist, insbesondere rechteckig.

9. Ultraschall-Schneidevorrichtung (1) nach einem der Ansprüche 1 bis 8,
**dadurch gekennzeichnet, dass** sie mehrere Ultraschallkonverter (6) aufweist, die jeweils über wenigstens einen Schallleiter (7) mit voneinander beabstandeten Schalleinkoppelstellen der Schneidklinge (8) verbunden sind.

10. Ultraschall-Schneidevorrichtung (1) nach einem der Ansprüche 1 bis 9, **dadurch gekennzeichnet, dass** der Schallleiter (7) einstückig mit der Schneidklinge (8) verbunden und vorzugsweise durch einen etwa U-förmig gekrümmten Messerschaft gebildet ist.

## Claims

1. Ultrasound cutting device (1) having at least one ultrasound converter (6) connected to a generator (2), at least one sound conductor (7) and at least one cutting blade (8), the sound conductor (7) being arranged between the ultrasound converter (6) and the cutting blade (8) and interconnecting the two, and the longitudinal centre axis of the sound conductor (7) extending along a line that deviates from a straight line, **characterized in that** the at least one generator (2) has means for running through a predetermined ultrasound frequency range in accordance with a sweep function.

2. Ultrasound cutting device (1) according to Claim 1, **characterized in that** it has a measuring arrangement for determining the power output by the generator (2) to the ultrasound converter (6), and **in that** the measuring arrangement is connected in terms of control to the means for running through the predetermined ultrasound frequency range in such a way that the ultrasound frequency range is selected to include the ultrasound frequency at which the generator (2) outputs its maximum power to the ultrasound converter (6).

3. Ultrasound cutting device (1) according to Claim 2, **characterized in that** the measuring arrangement is connected in terms of control to the means for running through the predetermined ultrasound frequency range in such a way that the generator (2) outputs its maximum power at an ultrasound frequency to the ultrasound converter (6) which is in the middle between the highest and the lowest frequency of the ultrasound frequency range through which the generator runs.

4. Ultrasound cutting device (1) according to one of Claims 1 to 3, **characterized in that** the cutting blade (8) is in the form of a planar element, and **in that** the sound conductor (7) is in the form of a guide rod, which is connected to the cutting blade (8) in a direction transverse to the area in which the cutting blade (8) extends.

5. Ultrasound cutting device (1) according to one of Claims 1 to 4, **characterized in that** the sound conductor (7) is annular, and **in that** a first end region of the sound conductor (7) is connected to the ultrasound converter (6) and a second end region diametrically opposite the first end region is connected to the cutting blade (8).

6. Ultrasound cutting device (1) according to Claim 5, **characterized in that** the cutting blade (8) is substantially cylindrical, and **in that** the sound conductor (7) is connected to the cutting blade (8) at an outer cylinder surface thereof.

7. Ultrasound cutting device (1) according to Claim 6, **characterized in that** the cutting blade (8) is circular-cylindrical or oval.

8. Ultrasound cutting device (1) according to Claim 6, **characterized in that** the cutting blade (8) is angular, in particular is rectangular.

9. Ultrasound cutting device (1) according to one of Claims 1 to 8, **characterized in that** it has a plurality of ultrasound converters (6), which are each connected via at least one sound conductor (7) to sound coupling-in locations of the cutting blade (8), which are spaced apart from one another.

10. Ultrasound cutting device (1) according to one of Claims 1 to 9, **characterized in that** the sound conductor (7) is connected integrally to the cutting blade (8) and is preferably formed by a knife shaft that is curved approximately in a U-shape.

## Revendications

1. Dispositif (1) de coupe par ultrasons, présentant
au moins un convertisseur (6) d'ultrasons raccordé à un générateur (2),
au moins un conducteur acoustique (7) et
au moins une lame de coupe (8),
le conducteur acoustique (7) étant disposé entre le convertisseur (6) d'ultrasons et la lame de coupe (8) et les reliant l'une à l'autre,
l'axe longitudinal central du conducteur acoustique (7) présentant une évolution différente d'une ligne droite,
**caractérisé en ce que**
le ou les générateurs (2) présentent des moyens permettant de balayer une plage prédéterminée de fréquences d'ultrasons en correspondance à une fonction de balayage.

2. Dispositif (1) de coupe par ultrasons selon la revendication 1, **caractérisé en ce qu'**il présente un dispositif de mesure qui détecte la puissance délivrée par le générateur (2) au convertisseur (6) d'ultrasons et **en ce que** le dispositif de mesure est en communication de commande avec le moyen de balayage de la plage prédéterminée de fréquences d'ultrasons, de telle sorte que la plage de fréquences d'ultrasons soit sélectionnée de manière à contenir la fréquence d'ultrasons à laquelle le générateur (2) délivre sa puissance maximale au convertisseur (6) d'ultrasons.

3. Dispositif (1) de coupe par ultrasons selon la revendication 2, **caractérisé en ce que** le dispositif de mesure est en communication de commande avec les moyens de balayage d'une plage prédéterminée de fréquences d'ultrasons de telle sorte que le générateur (2) délivre sa puissance maximale au convertisseur d'ultrasons (6) à une fréquence d'ultrasons située au milieu entre la fréquence la plus élevée et la fréquence la plus basse de la plage balayée lors d'un balayage en fréquence.

4. Dispositif (1) de coupe par ultrasons selon l'une des revendications 1 à 3, **caractérisé en ce que** la lame de coupe (8) est configurée comme élément plat et **en ce que** le conducteur acoustique (7) est configuré comme barreau de guidage raccordé transversalement à la surface dans laquelle s'étend la lame de coupe (8) et raccordé à cette dernière.

5. Dispositif (1) de coupe par ultrasons selon l'une des revendications 1 à 4, **caractérisé en ce que** le conducteur acoustique (7) a une configuration annulaire, **en ce qu'**une première partie d'extrémité du conducteur d'ultrasons (7) est raccordée au convertisseur (6) d'ultrasons et **en ce qu'**une deuxième partie d'extrémité diamétralement opposée à la première partie d'extrémité est raccordée à la lame de coupe (8).

6. Dispositif (1) de coupe par ultrasons selon la revendication 5, **caractérisé en ce que** la lame de coupe (8) a une configuration essentiellement cylindrique et **en ce que** le conducteur acoustique (7) est raccordé à la lame de coupe (8) par la surface d'enveloppe cylindrique de cette dernière.

7. Dispositif (1) de coupe par ultrasons selon la revendication 6, **caractérisé en ce que** la lame de coupe (8) a une configuration en cylindre circulaire ou en cylindre ovale.

8. Dispositif (1) de coupe par ultrasons selon la revendication 6, **caractérisé en ce que** la lame de coupe (8) a une configuration polygonale et en particulier rectangulaire.

9. Dispositif (1) de coupe par ultrasons selon l'une des revendications 1 à 8, **caractérisé en ce qu'**il présente plusieurs convertisseurs (6) d'ultrasons, chacun raccordé par au moins un conducteur acoustique (7) à des emplacements d'accouplement acoustique, disposés à distance les uns des autres, de la lame de coupe (8).

10. Dispositif (1) de coupe par ultrasons selon l'une des revendications 1 à 9, **caractérisé en ce que** le conducteur acoustique (7) est raccordé d'une seule pièce à la lame de coupe (8) et est formé de préférence par un arbre de couteau cintré sensiblement en forme de U.
